# EUROPÄISCHE PATENTANMELDUNG

(11) **EP 1 042 996 A2**
(43) Veröffentlichungstag der Anmeldung: **11.10.2000**
(21) Anmeldenummer: 00107359.2
(22) Anmeldetag: 05.04.2000
(51) Int. Cl.: A61F 2/06, A61M 25/00

(54) **Stentvorrichtung**

(30) Priorität: 09.04.1999 DE 19916060
(71) Anmelder: B. BRAUN MELSUNGEN AG, 34212 Melsungen (DE)
(72) Erfinder: Frimberger, Eckart, Dr., 80804 München (DE)
(74) Vertreter: Selting, Günther, Dipl.-Ing.

(57) **Zusammenfassung**

Die Stentvorrichtung weist einen rohrförmigen Stent (10) und einen Schubschlauch (18) zum Vorschieben des Stents (10) auf. Durch Stent (10) und Schubschlauch (18) kann ein Führungsstrang (17) geschoben werden. Zum Kuppeln des Stents an den Schubschlauch ist der Stent mit einem Faden (26) versehen, der in eine seitlich offene Aufnahme (25) des Schubschlauchs (18) eingelegt werden kann und dann durch den Führungsstrang (17) blockiert wird. Auf dem Führungstrang (17) erfolgt die Plazierung des Stents (10) in einem Körperkanal, wobei der Stent vorgeschoben und auch zurückgezogen werden kann. Nach Erreichen der korrekten Lage wird der Führungsstrang (17) herausgezogen und dann der Schubschlauch (18) entfernt. Der Faden (26) ist für ein Extraktionswerkzeug erreichbar, wenn der Stent (10) nachträglich herausgezogen oder in seiner Lage verändert werden soll.

## Beschreibung

Die Erfindung betrifft eine Stentvorrichtung zum Offenhalten von Körperkanälen, insbesondere des Gallenganges.

Es ist bekannt, zur Drainage von Körperhohlräumen eine Stentvorrichtung zu benutzen, bei der ein als Drainagerohr wirkender Stent auf einem Führungsstrang in den Patientenkörper eingeführt wird. In DE 38 31 652 C2 ist eine Stentvorrichtung beschrieben, die einen rohrförmigen Stent für die Drainage des Nierenhohlsystems aufweist. Dieser Stent wird über einen Führungsstrang eingeführt und nach dem Verlegen wird der Führungsstrang herausgezogen. Ferner ist ein als Pusher bezeichneter Schubschlauch über den Führungsstrang geschoben. An dem distalen Ende des Schubschlauchs ist das proximale Ende des Stents abgestützt. Damit nicht nur Schubkräfte, sondern auch Zugkräfte vorn Schubschlauch auf den Stent übertragen werden, ist eine Kupplungsvorrichtung vorhanden. Diese besteht aus zwei zueinander komplementären Teilen am Stent und am Schubschlauch, die sich gegenseitig übergreifen und durch den hindurchgesteckten Führungsstrang verriegelt sind, so daß sie bei eingeführtem Führungsstrang nicht gelöst werden können. Damit ist es möglich, den Stent sehr genau zu plazieren, wobei der Stent auch vor dem Abkuppeln wieder ein Stück zurückgezogen werden kann.

In DE 42 33 514 C1 ist ein Ureterkatheter beschrieben, der ebenfalls über einem Führungsstrang verlegt wird, wobei zusätzlich ein Vorschubschlauch vorgesehen ist. Der Katheter und der Vorschubschlauch sind durch eine lösbare Kupplungsvorrichtung verbunden, die durch den Führungsstrang arretiert wird.

Ein Körperkanal mit besonders schwierigem Zugang ist der Gallengang. Der Gallengang leitet die in der Leber produzierte Galle in den Zwölffingerdarm. An der Mündungsstelle befindet sich eine Engstelle, die als Papille oder Sphinkter bezeichnet wird und eine Ventilfunktion hat. Durch den Gallengang wird Gallenflüssigkeit, die in der Leber produziert worden ist, in den Darm eingeführt. Wenn der Gallengang zugesetzt ist, wird zum Offenhalten ein Stent eingeführt, bei dem es sich um eine rohrförmige Prothese handelt, durch die die Gallenflüssigkeit in den Darm geleitet wird. Das Einführen des Stents erfolgt in der Regel mit einem speziellen Endoskop, aus dessen Distalende der Stent seitlich herausgeschoben wird. Dabei kann es vorkommen, daß der Stent zu tief in den Gallengang eingeführt wurde oder sich nach dem Einführen verschiebt und im Gallengang verschwindet. Ferner kann es vorkommen, daß der Stent nach seiner Verlegung verstopft. In solchen Fällen ist es erforderlich, den Stent endoskopisch wieder zu entfernen oder eine Lagekorrektur vorzunehmen.

Der Erfindung liegt die Aufgabe zugrunde, eine Stentvorrichtung zu schaffen, die einerseits für die Verlegung des Stents in einem Körperkanal geeignet ist und andererseits auch die Möglichkeit späterer Lagekorrekturen oder einer Extraktion des Stents ermöglicht.

Die Lösung dieser Aufgabe erfolgt erfindungsgemäß mit den im Patentanspruch 1 angegebenen Merkmalen.

Bei der erfindungsgemäßen Stentvorrichtung weist die Kupplungsvorrichtung einen Faden auf, der die Verbindung mit dem Schubschlauch herstellt. Die Kupplungsvorrichtung wird durch den Führungsstrang verriegelt und beim Herausziehen des Führungsstranges entriegelt. Unter einem Führungsstrang ist hier ein Führungsdraht und auch ganz allgemein eine Führungssonde, beispielsweise aus Kunststoff bestehend, zu verstehen. Im entriegelten Zustand kann der Schubschlauch zurückgezogen werden, während der Stent in dem Körperkanal verbleibt. Am proximalen Ende des Stents befindet sich dann noch der Faden. Sollte sich herausstellen, daß der Stent in dem Körperkanal zu weit vorgeschritten ist, kann er durch Ziehen an dem Faden zurückgezogen werden. Auf diese Weise sind sowohl unmittelbar nach der Stentverlegung als auch zu einem späteren Zeitpunkt Lagekorrekturen auf einfache Weise möglich, indem ein Instrument, das z.B. eine Zange zum Ziehen an dem Faden aufweist, in den Körper eingeführt wird. Auch eine spätere Entfernung des Stents ist auf diese Weise ohne Probleme möglich.

Die erfindungsgemäße Stentvorrichtung eignet sich insbesondere für einen Gallengangstent. Der Gallengang ist ein mit invasiven Methoden nur sehr schwer zugänglicher Körperkanal. Der am proximalen Ende des Stents befindliche Faden ist nach der Verlegung des Stents vom Zwölffingerdarm her gut zugänglich und kann daher mit einem Greifinstrument, beispielsweise einer Zange, vom Darm aus gut erreicht werden.

Der Stent bildet ein langgestrecktes Drainagerohr mit einer Länge von etwa 4-18 cm zur Überbrückung von Verengungen (Stenosen) oder Verschlüssen, vorzugsweise in einem Gallengang. Zweckmäßigerweise ist der Stent zur Fixierung mit Widerhaken versehen. Ein solcher Widerhaken und/oder eine den Widerhaken tragende Manschette wird zweckmäßigerweise als Rückhalteelemente für den Faden benutzt. Der Faden hat typischerweise eine Länge von etwa 2-10 cm.

Vorzugsweise bildet der Faden eine Fadenschlaufe. Dies ermöglicht eine einfache und haltbare Kupplung mit dem Schubschlauch und ein einfaches Ergreifen zum Ausführen von Lagekorrekturen mit einem Instrument, das z.B. einen Haken aufweist.

Im folgenden werden Ausführungsbeispiele der Erfindung unter Bezugnahme auf die Zeichnungen näher erläutert.

Es zeigen:
- Fig. 1: eine Seitenansicht eines für die Gallengangdrainage bestimmten Stents,
- Fig. 2: in vergrößertem Maßstab eine Seitenansicht der Kupplungsstelle zwischen Stent und Schubschlauch,
- Fig. 3: eine Darstellung der Enden von Stent und Schubschlauch nach dem Entfernen des Führungsstranges,
- Fig. 4: ein zweites Ausführungsbeispiel der Stentvorrichtung,
- Fig. 5: eine Darstellung der Enden von Stent und Schubschlauch des zweiten Ausführungsbeispiels nach dem Entfernen des Führungsstranges und
- Fig. 6: das Ende eines Schubschlauchs gemäß einem dritten Ausführungsbeispiel in Seitenansicht.

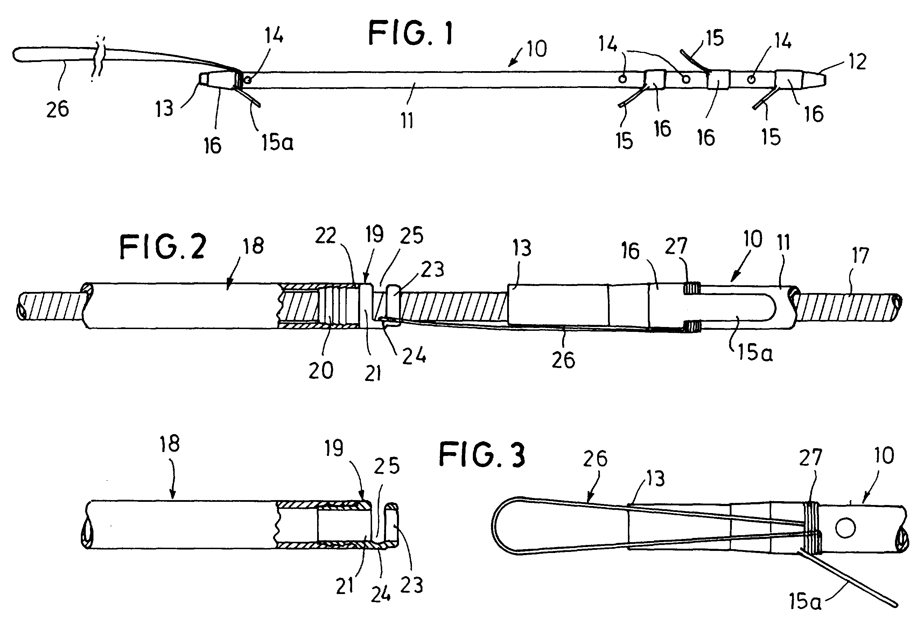

In Fig. 1 ist ein Stent 10 für die Gallengangdrainage dargestellt. Dieser Stent besteht aus einem langgestreckten Schlauch 11 aus halbstarrem Kunststoff, der biegbar ist, jedoch einen großen Widerstand gegen Zusammendrücken bietet. Der Schlauch 11 hat eine Länge von etwa 4-18 cm und einen Innendurchmesser von mindestens etwa 1 mm. Am distalen Ende 12 und am proximalen Ende 13 ist der Schlauch 11 offen. In der Nähe des distalen Endes 12 sind seitliche Löcher 14 vorgesehen und ein weiteres seitliches Loch 14 befindet sich in der Nähe des proximalen Endes 13.

Der Stent 10 hat ferner mehrere schräg nach hinten abstehende Widerhaken 15, die jeweils von einer kreisförmigen Manschette 16 getragen werden, welche den Schlauch 11 eng umgibt. Am proximalen Ende 13 ist ebenfalls eine Manschette 16 mit Widerhaken 15a vorgesehen, wobei dieser Widerhaken jedoch schräg nach vorne gerichtet ist.

Fig. 2 zeigt den proximalen Endbereich des Stents 10, wobei durch das Lumen des Stents ein Führungsstrang in Form eines Führungsdrahts 17 hindurchgeführt ist. Der Führungsdraht 17 bildet eine Einführhilfe für die Implantation des Stents. Auf den Führungsdraht 17 ist ferner ein Schubschlauch 18 aufgeschoben, der mit seinem distalen Ende gegen das proximale Ende 13 des Stents 10 drücken kann, um den Stent relativ zu dem Führungsdraht 17 in Längsrichtung zu verschieben. Sowohl der Stent 10 als auch der Schubschlauch 18 sind auf dem Führungsdraht 17 in Längsrichtung verschiebbar. An dem distalen Ende des Schubschlauches 18 befindet sich ein Kupplungselement 19 mit einer in den Schubschlauch eingeschobenen Rohrmuffe 20 und einem Ringteil 21, das sich an dem Ende 22 des Schubschlauches 18 abstützt. Parallel zu dem Ringteil 21 ist ein weiteres Ringteil 23 mit axialem Abstand angeordnet. Die beiden Ringteile 21 und 23 sind durch eine axiale Brücke 24 miteinander verbunden. Zwischen den Ringteilen 21 und 23 befindet sich eine Aufnahme 25 in Form eines seitlich offenen Schlitzes. Dieser Schlitz erstreckt sich über einen Umfangswinkel von mehr als 180°, vorzugsweise von mehr als 270°, so daß die Brücke 24 einen Umfangswinkel von weniger als 90° einnimmt.

An dem Stent 10 ist eine Fadenschlaufe 26 befestigt. Diese weist einen Fadenwickel 27 auf, der um den Schlauch 11 herumgewickelt ist, und zwar unterhalb des Widerhakens 15a, so daß der Widerhaken eine Bewegung des Fadenwickels 27 zum proximalen Ende 13 hin verhindert. Von dem Fadenwickel 27 gehen zwei Schlaufenbeine ab, die die geschlossene Fadenschlaufe 26 bilden. Die Fadenschlaufe 26 erstreckt sich über das proximale Ende 13 des Stents 10 hinaus.

Gemäß Fig. 3 wird zunächst die Fadenschlaufe 26 in die Aufnahme 25 am Schubschlauch 18 von der Seite her eingesetzt, bis sie gegen die Brücke 24 stößt. In diesem Zustand haben der Stent und der Schubschlauch 18 einen axialen Abstand voneinander. Dann wird der Führungsdraht 17 durch den Schubschlauch 18 und den Stent 10 geschoben, so daß er die in Fig. 2 dargestellte Position einnimmt. Dadurch wird die Fadenschlaufe 26 in der Aufnahme 25 vom Führungsdraht 17 verriegelt. Der Schubschlauch 18 kann relativ zu dem Führungsdraht 17 vorgeschoben werden, um dabei den Stent 10 zu verschieben. Ferner kann der Schubschlauch 18 (nach links) zurückgezogen werden, wobei er den Stent 10 über die Fadenschlaufe 26 mitnimmt.

Nachdem der Stent 10 in dem Körperkanal plaziert worden ist, wird der Führungsdraht 17 aus dem Stent 10 und dem Schubschlauch 18 herausgezogen. Dabei wird die Fadenschlaufe 26 freigegeben, so daß sie die Aufnahme 25 verlassen kann. Die Fadenschlaufe 26 steht nun frei von dem proximalen Ende 13 des Stents 10 ab. Sie kann mit einem Greifinstrument oder einem Haken ergriffen werden, um bei Bedarf den Stent aus dem Körperkanal herauszuziehen.

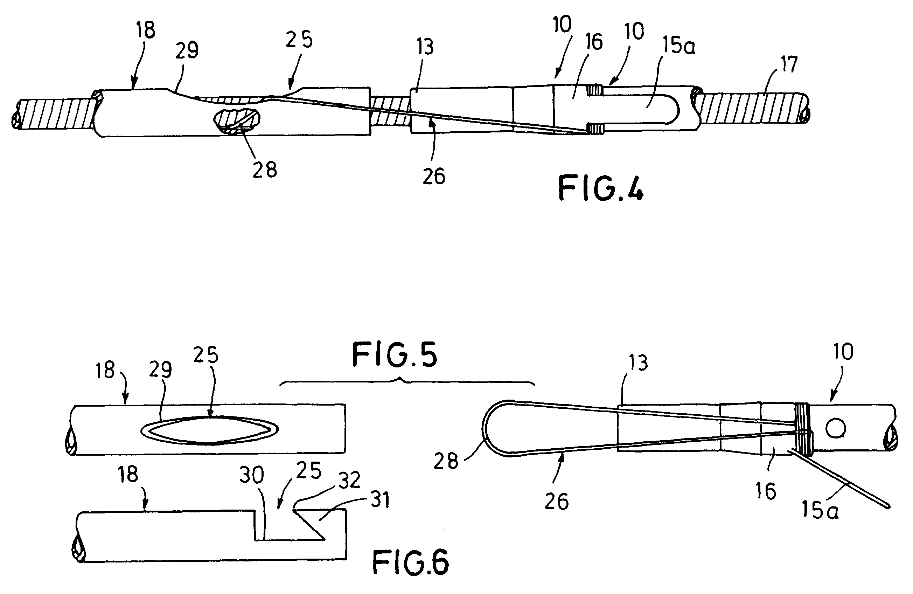

In den Fign. 4 und 5 ist ein zweites Ausführungsbeispiel dargestellt, bei dem die Aufnahme 25 aus einer längslaufenden Öffnung 29 in der Seitenwand des Schubschlauchs 18 besteht. Die Fadenschlaufe 26 ist mit beiden Schlaufenbeinen in die Öffnung 29 eingelegt, so daß die Schlinge 28 sich im Innern des Schubschlauchs 18 befindet und dort von dem Führungsdraht 17 bedeckt wird. Die Schlinge 28 liegt also auf der der Öffnung 29 gegenüberliegenden Seite des Lumens und wird von dem Führungsdraht 17 arretiert. Nach dem Herausziehen des Führungsdrahtes 17 wird die Fadenschlaufe 26 frei, so daß der Schubschlauch 18 entfernt werden kann, während der Stent 10 am Platz verbleibt. Die Öffnung 29 hat eine Breite, die sich über weniger als 180° des Umfangs des Schubschlauchs erstreckt und vorzugsweise über etwa 90°. Die Länge der Öffnung 29 ist größer als die Breite.

In Fig. 6 ist das Ende eines Schubschlauches 18 gemäß einem dritten Ausführungsbeispiel dargestellt. Der nicht gezeigte Stent ist wie bei den ersten beiden Ausführungsbeispielen ausgebildet. Die Aufnahme 25 ist hier als in den Schubschlauch 18 eingebrachte widerhakenförmige Öffnung 30 vorgesehen. Die Öffnung 30 ist in Seitenansicht keilförmig. Dadurch wird in dem Schubschlauch 18 ein zungenförmiger Widerhaken 31 ausgebildet, dessen Spitze 32 axial von dem Stent weg nach hinten gerichtet ist. Die Zunge beginnt an den Außenkanten der Öffnung 30 und läuft in der Mitte zusammen, um die Spitze 32 zu bilden.

Die nicht dargestellte Fadenschlaufe ist zunächst in die Öffnung 30 eingelegt, so daß sie den Schubschlauch 18 durchquert. Durch Herausziehen des Führungsdrahtes wird die Fadenschlaufe freigegeben, wenn der Schubschlauch 18 ein Stück weiter vorgeschoben wird. Der Schubschlauch 18 kann auch als Greifhaken zum Wiedererfassen und Zurückziehen des Stents benutzt werden. Hierbei gleitet die Fadenschlaufe des Stents entlang der Schräge des Widerhakens 31 nach unten, so daß der Faden in dem Widerhaken festgezogen wird.

## Patentansprüche

1. Stentvorrichtung zum Offenhalten von Körperkanälen, insbesondere des Gallenganges, mit
- einem rohrförmigen Stent (10),
- einem Führungsstrang (17), über den der Stent (10) schiebbar ist,
- einem über den Führungsstrang (17) schiebbaren Schubschlauch (18) zum Vorschieben des Stents relativ zum Führungsstrang,
- und einer Kupplungsvorrichtung zum lösbaren Verbinden des Stents (10) mit dem Schlauch,
**dadurch gekennzeichnet,**
daß die Kupplungsvorrichtung einen am Stent (10) befestigten Faden aufweist, der in eine seitlich offene Aufnahme (25) am Schubschlauch (18) einführbar ist und von dem eingeschobenen Führungsstrang (17) blockiert wird.

2. Stentvorrichtung nach Anspruch 1, dadurch gekennzeichnet, daß der Faden eine Fadenschlaufe (26) bildet.

3. Stentvorrichtung nach Anspruch 1 oder 2, dadurch gekennzeichnet, daß die Aufnahme (25) in einem am Schubschlauch (18) befestigten Kupplungselement (19) ausgebildet ist, welches die Aufnahme (25) als querverlaufenden Schlitz aufweist.

4. Stentvorrichtung nach Anspruch 1 oder 2, dadurch gekennzeichnet, daß die Aufnahme (25) am Schubschlauch (18) gebildet ist und eine längslaufende Öffnung (29) in der Seitenwand des Schubschlauchs (18) aufweist.

5. Stentvorrichtung nach einem der Ansprüche 1-4, dadurch gekennzeichnet, daß an dem Schubschlauch (18) eine Öffnung (30) gebildet ist, die in Seitenansicht die Form eines Widerhakens (31) mit proximal gerichteter Spitze (32) aufweist.

6. Stentvorrichtung nach einem der Ansprüche 1-5, dadurch gekennzeichnet, daß der Stent (10) an seinem Umfang Widerhaken (15,15a) aufweist, und daß die Widerhaken (15a) und/oder die den Widerhaken tragende Manschette (16) als Rückhalteelement für den Faden dient.
